(19) **Europäisches Patentamt**
**European Patent Office**
**Office européen des brevets**

(11) **EP 3 977 934 A1**

(12) **EUROPEAN PATENT APPLICATION**

(43) Date of publication:
**06.04.2022 Bulletin 2022/14**

(21) Application number: **20290069.2**

(22) Date of filing: **02.10.2020**

(51) International Patent Classification (IPC):
***A61B 6/00*** (2006.01)

(52) Cooperative Patent Classification (CPC):
**A61B 6/481; A61B 6/486; A61B 6/504; A61B 6/507; A61B 6/5217**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicant: **Koninklijke Philips N.V.**
**5656 AG Eindhoven (NL)**

(72) Inventors:
• **FLORENT, Raoul**
  **5656 AE Eindhoven (NL)**
• **VAN DER HORST, Arjen**
  **5656 AE Eindhoven (NL)**
• **THIS, Alexandre**
  **5656 AE Eindhoven (NL)**

(74) Representative: **Philips Intellectual Property & Standards**
**High Tech Campus 52**
**5656 AG Eindhoven (NL)**

(54) **SYSTEMS AND METHODS FOR PERFORMING BLOOD FLOW VELOCITY MEASUREMENTS**

(57)     The invention provides a processor for use in a system for obtaining a blood flow velocity measurement based on an angiogram obtained from a subject. The angiogram is representative of a vessel including a contrast agent therein, and wherein the contrast agent has been provided to the vessel in bursts at a given average frequency, thereby providing contrast data within the angiogram

    The processor generates a velocity curve representing the blood flow velocity within the vessel over the cardiac cycle based on an estimated period of the cardiac cycle, an estimated instantaneous blood flow velocity of each angiogram image frame, a calculated confidence value of each angiogram image frame and the estimated point within the cardiac cycle represented by each angiogram image frame.

FIG. 3

## Description

FIELD OF THE INVENTION

The invention relates to the field of blood flow velocity measurements, and more specifically to the field of blood flow velocity measurements using angiograms.

BACKGROUND OF THE INVENTION

Guiding the treatment of cardiovascular and peripheral vascular disease based on hemodynamic measurements typically improves the final outcome of the treatment. In particular, with regard to the coronary arteries, clinical trials have shown that decision making based on blood pressure and blood flow measurements improves the clinical outcome compared to trials based on angiography alone, as discussed in Pijls, Nico H. J., et al. "Fractional Flow Reserve Versus Angiography for Guiding Percutaneous Coronary Intervention in Patients With Multivessel Coronary Artery Disease." Journal of the American College of Cardiology, vol. 56, no. 3, Elsevier Inc., July 2010, pp. 177-84, doi:10.1016/j.jacc.2010.04.012.

Blood flow measurements are particularly valuable in the case of non-obstructive coronary artery disease, ie. angina complaints without any visible obstructions in the larger arteries. Furthermore, blood flow measurements in treating other pathologies, such as peripheral vascular disease, may help to quantify the effect of atherosclerosis on the blood supply to the feet and define the end point of endovascular treatment. Additionally, in the field of oncology interventions, blood flow monitoring during embolization interventions may be helpful in assessing the degree of embolization and deciding when to stop treatment in order to prevent embolization of healthy tissue, for example in trans arterial chemoembolization (TACE). In the field of hypertension management, renal blood flow may be a predictor of the effectiveness of renal denervation procedures. Further, in interventional neurology, blood flow measurements may indicate whether a deployed flow diverter has a positive impact on the hemodynamic behavior observed within the treated aneurysm.

In summary, blood flow velocity is an important metric in a wide variety of different procedures and treatments. There are several methods available for assessing blood flow velocity. For example, guidewires with a Doppler ultrasound sensor or temperature sensors are available on the market for the assessment of blood flow. A disadvantage of these methods is the cost of the devices and the need for the devices to be inserted into the arteries, which carries an additional risk of complications. External ultrasound may also be used to assess blood flow; however, a highly experienced sonographer is needed to acquire accurate measurements in this way. Additionally, coronary and renal arteries are difficult to image using external ultrasound due to the location of the vessels inside the body.

Angiograms may also be used to assess blood blow. The use of angiograms is appealing because no additional hardware is required and the assessment of blood flow may form part of an existing X-Ray interventional workflow.

Among the quantitative approaches for estimating blood flow based on an angiogram, one of the most commonly adopted approaches utilizes bolus front propagation, as described in Khanmohammadi et al, Automatic Estimation of Coronary Blood Flow Velocity Step 1 for Developing a Tool to Diagnose Patients With Micro-Vascular Angina Pectoris, Frontiers in Cardiovascular Medicine, Jan. 2019. Another commonly employed approach is based on blood pulsatility, as described in Bonnefous and al, Quantification of arterial flow using digital subtraction angiography, Med. Phys. 39 (10), October 2012. Further there are blood flow measurement methods based on a pulsed contrast signal in which the injection of a contrast agent is not continuous but pulsed, such as those methods described in Holdsworth, D. W., et al. "Quantitative Angiographic Blood-Flow Measurement Using Pulsed Intra-Arterial Injection." Medical Physics, vol. 26, no. 10, Oct. 1999, pp. 2168-75, doi:10.1118/1.598733 and Shaw, C. G., and D. B. Plewes. "Pulsed-Injection Method for Blood Flow Velocity Measurement in Intraarterial Digital Subtraction Angiography." Radiology, vol. 160, no. 2, Aug. 1986, pp. 556-59, doi:10.1148/radiology.160.2.3523598.

Looking specifically to those methods for deriving blood flow velocity from angiograms, the existing methods suffer from a number of drawbacks as discussed below.

The methods based on bolus front propagation suffer from the fact that it is only possible to estimate blood flow velocity during the temporal interval that corresponds to the observed front traversal, i.e. the time the bolus front takes to cross the observed vessel segment. If the blood flow velocity is high, the time taken by the bolus front to cross the visible vessel segment may be very limiting and may only provide information during a fraction of the cardiac cycle. As blood flow velocity varies throughout the cardiac cycle, this may lead to strong under or over evaluation of the average blood velocity.

The methods based on blood pulsatility are able to determine the blood flow velocity over the full cardiac cycle, but are very sensitive to the contrast to noise ratio in the angiogram. This contrast to noise ratio sensitivity makes the use of such methods problematic, if not impossible. Further, these methods require large integration distances and so cannot be applied to short vessels, such as the renal arteries.

As mentioned above, some methods utilize a pulsed contrast agent injection, in an attempt to alleviate the limitation of bolus front propagation by artificially creating several bolus fronts. For instance, the methods described in Shaw, C. G., and D. B. Plewes. "Pulsed-Injection Method for Blood Flow Velocity Measurement in Intraarterial Digital Subtraction Angiography." Radiology, vol. 160, no. 2, Aug. 1986, pp. 556-59, doi:10.1148/radiology.160.2.3523598 do not use the bolus front, but rather compute an average velocity based on the distances between the contrast pulses. These methods rely on very controlled, high frequency injections of contrast agent, for which highly specialized equipment is required. Such injection conditions are very demanding and not easily applicable in practice. Furthermore, this method assumes that at least two pulses are visible at a given instant on the image, which limits the application of the method to vessels that are sufficiently long.

The methods described in Holdsworth, D. W., et al. "Quantitative Angiographic Blood-Flow Measurement Using Pulsed Intra-Arterial Injection." Medical Physics, vol. 26, no. 10, Oct. 1999, pp. 2168-75, doi:10.1118/1.598733 generates an instantaneous velocity estimation based on the pulse front propagation using high frequency pulse injections. The high frequency component helps to increase the accuracy of the measurement, but requires highly specialized injection equipment as discussed above.

There is therefore a need for a means of determining the blood flow velocity within a vessel of any length using an angiogram and without requiring any additional hardware or specialized equipment compared to a conventional angiogram.

SUMMARY OF THE INVENTION

According to examples in accordance with an aspect of the invention, there is provided a processor for use in a system for obtaining a blood flow velocity measurement based on an angiogram obtained from a subject, wherein the processor is adapted to:

> obtain an angiogram of a vessel of the subject, the angiogram comprising a plurality of angiogram image frames, wherein the angiogram is representative of the vessel including a contrast agent therein, and wherein the contrast agent has been provided to the vessel in bursts, thereby providing contrast data within the angiogram;
> for each angiogram image frame of the angiogram that includes contrast data:
>
>> estimate an instantaneous blood flow velocity based on the contrast data;
>> calculate a confidence value of the angiogram image frame based on the contrast data; and
>> estimate a point within a cardiac cycle represented by the angiogram image frame;
>
> estimate a period of the cardiac cycle; and
> generate a velocity curve representing the blood flow velocity within the vessel over the cardiac cycle based on the period of the cardiac cycle, the estimated instantaneous blood flow velocity of each angiogram image frame, the confidence value of each angiogram image frame and the point within the cardiac cycle represented by each angiogram image frame.

The invention provides a means of deriving accurate blood flow velocity measurements from an angiogram of a vessel which can be implemented using only standard X-ray imaging equipment.

Each point on the velocity curve is generated based on a combination of the estimated instantaneous blood flow velocity and the confidence value of the angiogram image frame associated with that point in time within the cardiac cycle.

As the instantaneous blood flow velocity derived from each angiogram image frame is combined with a confidence value for each image frame, determined based on the contrast data, the frequency at which the contrast agent needs to be provided to the vessel may be lower than if the blood flow velocity was to be derived without the use of a confidence value. In this way, low frequency contrast agent injections, which do not require specialist hardware, may be used to provide the contrast agent to the vessel.

Accordingly, an accurate blood flow velocity measurement may be derived from the angiogram using conventional X-ray imaging hardware. Further, as the contrast data is utilized for confidence value calculations in addition to blood flow velocity measurements, it is possible to derive blood flow velocity measurements from an angiogram of a short vessel, which would not otherwise be possible.

In an embodiment, the frequency at which the bursts of contrast agent have been provided to the vessel is desynchronized with the period of the cardiac cycle. In this way, contrast agent wave fronts within the angiogram of the vessel span different phases of the cardia cycle, thereby increasing the accuracy of the final velocity curve.

In some embodiments, the bursts are separated by:

> regular intervals; or
> irregular intervals.

In other words, the bursts may be periodic or non-periodic. In this way, any injection pattern may be utilized to achieve an optimal measurement of the velocity curve of the vessel. The non-periodic bursts may alternatively be characterized by a predetermined average frequency of bursts, whereas for periodic bursts the frequency of bursts is the average frequency of bursts.

In an embodiment, the contrast agent is provided to the vessel in bursts at a predetermined average frequency, which in an example may be less than or equal to 15Hz.

In this way, the blood flow velocity curve may be derived in an accurate manner using a contrast agent provided at a low frequency.

In an embodiment, calculating the confidence value is further based on a noise level of the angiogram image frame.

In order to obtain an accurate instantaneous blood flow velocity measurement from the angiogram image frame, the contrast data should feature a distinguishable pattern that can be recognized by the processor and said pattern should have a higher energy than the noise energy within the angiogram image frame. Thus, by accounting for the signal-to-noise ratio of the angiogram image frame, the accuracy of the instantaneous blood flow velocity, and the velocity curve, may be increased.

In an embodiment, calculating the confidence value is further based on the estimated instantaneous blood flow velocity.

In this way, the accuracy of the confidence value may be increased. In particular, using the blood flow velocity measurements to compute the confidence value is advantageous because the confidence value may be further refined if any of the velocity measurements are outside of a known range of possible values. Further, the confidence value may be refined if the velocity measurements appear to be unstable along the vessel.

In an embodiment, estimating the period of the cardiac cycle based on one or more of:

> the angiogram; and
> ECG data.

In an embodiment, the processor is further adapted to identify, based on the velocity curve, one or more of:

> an optimal injection pattern for the contrast agent to be provided to the vessel; and
> an optimal injection duration for the contrast agent to be provided to the vessel.

In this way, a contrast agent injection pattern may be obtained which provides a fully populated velocity curve, thereby increasing the accuracy of a subsequently obtain velocity curve. In other words, based on the shape of an obtained velocity curve, an injection pattern for a contrast agent may be derived which results in an instantaneous blood flow velocity measurement and associated confidence value to be obtained in a desired distribution throughout the cardiac cycle.

In an embodiment, the processor is further adapted to:

> obtain pressure data from the vessel; and
> calculate a vascular impedance based on the pressure data and the velocity curve.

In this way, additional information regarding the status of the vessel may be derived based on the velocity curve obtained from the angiogram.

In an embodiment, the processor is further adapted to:

> obtain a second angiogram of a vessel of the subject, the second angiogram having been acquired after a stimulus was provided to the subject, the second angiogram comprising a plurality of second angiogram image frames, wherein the second angiogram is representative of the vessel including a contrast agent therein, and wherein the contrast agent has been provided to the vessel in bursts, thereby providing contrast data within the angiogram;
> for each second angiogram image frame of the angiogram that includes contrast data:
>
> > estimate a stimulated instantaneous blood flow velocity based on the second angiogram image frame;
> > calculate a stimulated confidence value of the second angiogram image frame based on the contrast data; and
> > estimate a point in time within a cardiac cycle represented by the second angiogram image frame;
>
> estimate a period of the cardiac cycle;
> generate a stimulated velocity curve for the cardiac cycle based on the period of the cardiac cycle, the estimated stimulated instantaneous blood flow velocity of each second angiogram image frame, the stimulated confidence value of each second angiogram image frame and the point in time within the cardiac cycle represented by or associated to each second angiogram image frame;
> compare the velocity curve and the stimulated velocity curve; and
> calculate a relative index based on the comparison.

In this way, the status of the vasculature may be determined based on a comparison between velocity curves obtained before and after a stimulus has been provided to the subject.

In an embodiment, the processor is further adapted to:

> determine a vessel diameter for on each angiogram image frame; and
> determine a blood flow volume based on the vessel diameters and the velocity curve.

In this way, additional information regarding the status of the vessel may be derived based on the velocity curve obtained from the angiogram.

According to examples in accordance with an aspect of the invention, there is provided a system for obtaining a blood flow velocity measurement based on an angiogram obtained from a subject, the system comprising:

a contrast agent injection device adapted to inject a contrast agent into a vessel of the subject in bursts provided according to any of the disclosed embodiments;

an X-ray imaging device adapted to obtain an angiogram of the vessel; and

a processor according to any of the respective disclosed embodiments.

In an embodiment, the system further comprises a feedback module adapted to identify an optimal injection pattern or an optimal injection duration for the contrast agent to be provided to the vessel by way of the contrast agent injection device based on the velocity curve.

In an embodiment, the contrast agent injection device is operated automatically based on the optimal injection pattern or the optimal injection duration identified by the feedback module.

In an embodiment, the contrast agent injection device is operated manually by a user, and wherein the system further comprises a display adapted to display information relating to the optimal injection pattern or the optimal injection duration to the user.

In an aspect of the invention a method of ascertaining a blood flow velocity is provided based on an angiogram obtained from a subject, wherein the method comprises the steps of:

obtaining an angiogram of a vessel of the subject, the angiogram comprising a plurality of angiogram image frames, wherein the angiogram is representative of the vessel including a contrast agent therein, and wherein the contrast agent has been provided to the vessel in bursts, thereby providing contrast data within the angiogram;

for each angiogram image frame of the angiogram that includes contrast data:

estimating an instantaneous blood flow velocity based on the contrast data;
calculating a confidence value of the angiogram image frame based on the contrast data; and
estimating a point in time within a cardiac cycle associated to the angiogram image frame;

estimating a period of the cardiac cycle; and
generating a velocity curve representing the blood flow velocity within the vessel over the cardiac cycle based on the period of the cardiac cycle, the estimated instantaneous blood flow velocity of each angiogram image frame, the confidence value of each angiogram image frame and the point in time within the cardiac cycle represented by or associated to each angiogram image frame.

In yet a further aspect of the invention a computer program is provided, wherein the computer program code means of the computer program are adapted, when said computer program run on a computer, to implement the method according to the invention on any of the systems according to the invention.

These and other aspects of the invention will be apparent from and elucidated with reference to the embodiment(s) described hereinafter.

BRIEF DESCRIPTION OF THE DRAWINGS

For a better understanding of the invention, and to show more clearly how it may be carried into effect, reference will now be made, by way of example only, to the accompanying drawings, in which:

Figure 1 shows a flow diagram illustrating the steps carried out by a processor according to an aspect of the invention;
Figure 2 shows a graph of blood flow velocity against time; and
Figure 3 shows a schematic representation of a system for obtaining a blood flow velocity measurement based on an angiogram.

DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

The invention provides a processor for use in a system for obtaining a blood flow velocity measurement based on an angiogram obtained from a subject. The angiogram is representative of a vessel including a contrast agent therein, and wherein the contrast agent has been provided to the vessel in bursts at a predetermined or given frequency, thereby providing contrast data within the angiogram.

For each angiogram image frame of the angiogram that contains contrast data, the processor is adapted to: estimate an instantaneous blood flow velocity based on the contrast data; calculate a confidence value of the angiogram image frame based on the contrast data; estimate a point within a cardiac cycle represented by the angiogram image frame; and estimate a period of the cardiac cycle.

The processor generates a velocity curve representing the blood flow velocity within the vessel over the cardiac cycle based on an estimated period of the cardiac cycle, an estimated instantaneous blood flow velocity of each angiogram image frame, a calculated confidence value of each angiogram image frame and the estimated point within the cardiac cycle represented by each angiogram image frame.

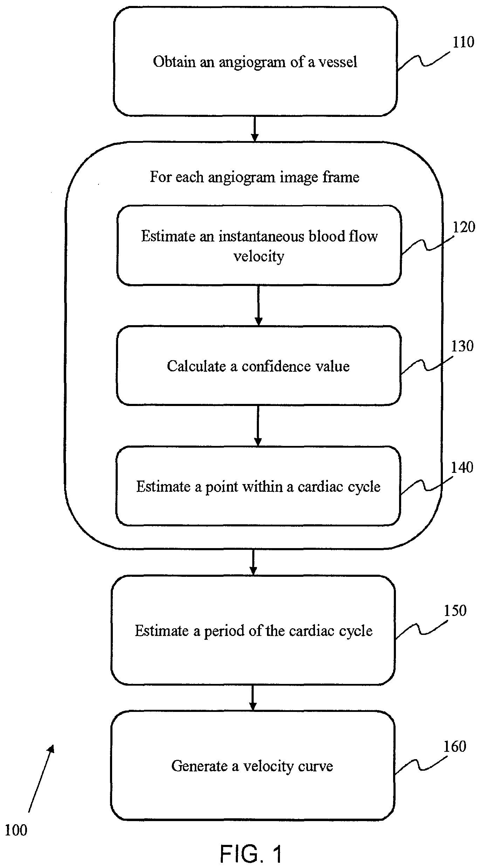

Figure 1 shows a flow diagram 100 illustrating the steps carried out by the processor when obtaining a blood flow velocity measurement based on an angiogram acquired from a subject.

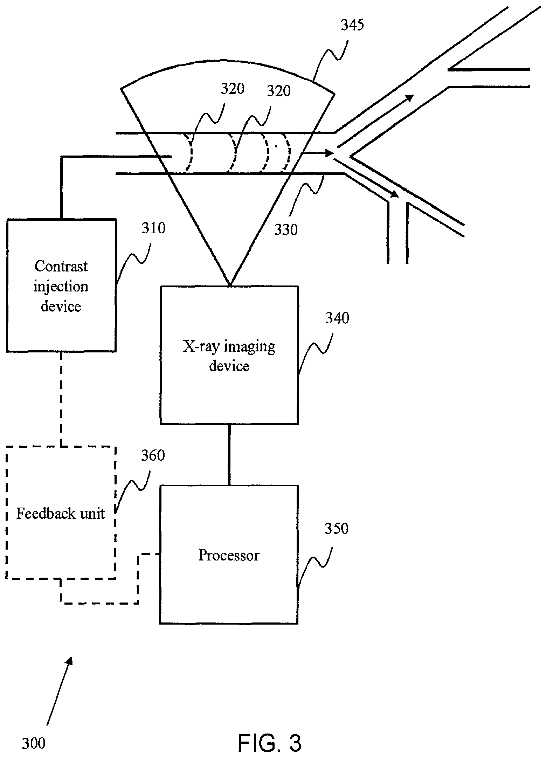

In step 110, an angiogram of a vessel of the subject is obtained. The angiogram comprises a plurality of individual angiogram image frames and is representative of a vessel that has been provided with a contrast agent in bursts at a predetermined or given average frequency, for example of less than 15Hz, thereby providing contrast data within the angiogram. Put another way, the angiogram is representative of a vessel including a contrast agent therein. The contrast agent may be provided to the vessel in any suitable manner, examples of which are discussed below with respect to Figure 3.

The given average frequency at which the bursts of contrast agent have been provided to the vessel may be desynchronized with the period of the cardiac cycle. By desynchronizing the frequency at which the bursts of contrast agent are provided to the vessel, the contrast agent wave fronts within the angiogram will span different phases of the cardiac cycle, meaning that the velocity measurements obtained in subsequent steps will provide a more complete picture of the blood flow velocity curve in the vessel over an entire cardiac cycle.

The bursts of contrast agent may be separated by regular intervals or irregular intervals. By providing the bursts at regular intervals, the velocity measurements will be evenly spaced across the velocity curve. By utilizing irregular intervals, regions of the velocity curve requiring more frequent measurement, such as regions having a high velocity gradient, may be measured more accurately. Accordingly, for simplifying the terminology used in the description, average frequency of bursts will be used for periodic and non-periodic bursts, wherein the non-periodic bursts are characterized by an average frequency of bursts, whereas for periodic bursts the frequency of bursts is in fact the average frequency of bursts.

The steps 120 to 140 are performed for each angiogram image frame of the angiogram including contrast data.

In step 120, an instantaneous blood flow velocity is estimated based on the contrast data. The estimation of the instantaneous blood flow velocity within the vessel may be performed using any suitable method, such as a correlation technique or an optical flow method.

In step 130, a confidence value of the angiogram image frame is calculated based on the contrast data.

Using the angiogram image frame, a confidence value, or a weight, is calculated based on the contrast information observed along the vessel of interest. The contrast information may include any information relating to the contrast agent within the vessel, such as: a front of the contrast agent; a spread of the contrast agent; a spacing between adjacent contrast agent fronts; a displacement of a contrast front between different angiogram image frames; and the like. The confidence value is related to the saliency, or prominence, of the contrast pattern along the vessel at a given instant, as represented by the contrast data. For example, if the contrast is constant along the entire vessel at time t, then there is no real pattern that can be exploited to deduce the speed of the movement of the contrast agent, and hence the blood flow velocity. Whereas, if at time t the contrast data features a well-marked step-edge aspect, this pattern might be identified at other points in time at different positions along the vessel, thereby providing information on the blood flow velocity in the vessel. The variance of the contrast data may be used for the confidence measurement, as the variance captures the energy of the non-constant component of a signal. Normalization of the contrast data may not be required; however, by normalizing the contrast data, a fixed level may be established above which a confidence value may be considered as describing a trustworthy sample.

By way of example, the confidence value may be calculated as:

$$R(t) = \frac{\sqrt{Var_x(C(t,x)) - N^2}}{N}$$

wherein: R(t) is the confidence value; C(t, x) is the contrast intensity at a given position, x, and time, t, $Var_x$ is the variance of C(t,x) along the x dimension; and N is the standard deviation of the noise level in the angiogram image frame.

In this example, the confidence value describes the contrast variance from which the noise variance is subtracted and then normalized by the noise level, N. The measurements are retained whenever the R(t) > $R_0$, where $R_0$ is a threshold value. Alternatively, the measurements can be combined using R(t) as a relative weight in this combination.

Put another way, calculating the confidence value may be based on a signal-to-noise ratio of the angiogram image frame, where the noise level is estimated in the angiogram image, and the signal is estimated in the same image but along the vessel of interest, as a measurable contrast or variance.

The confidence value provides a means for identifying the instantaneous blood flow velocity measurements where the blood flow velocity estimation is the most trustworthy. For example, if the contrast data does not show any variation along the vessel, it is not possible to track the movement of the contrast agent over time.

The contrast data must feature a distinguishable pattern in order to identify the instantaneous blood flow velocity and said pattern must have a higher energy than the noise energy. Put another way, the contrast data should not be buried in noise.

Further, the calculation of the confidence value may also be based on the estimated instantaneous blood flow velocity. Using the instantaneous blood flow velocity measurement in the computation of the confidence values may help to further refine the confidence value. For example, if the blood flow velocity measurement is clearly outside of a known range, defined based on existing clinical knowledge, these measurements may be automatically discarded as being erroneous. Further, if the blood flow velocity measurements appear to be unstable along the vessel segment, the confidence value may be lowered for that image frame based on the mass conservation principle, which means that, in a straight tube without bifurcations, the flow should be constant along the tube.

In step 140, a point within a cardiac cycle represented by the angiogram image frame is estimated. Put another way, each angiogram image frame is associated with a point in time within the cardiac cycle period. The position along the cardiac cycle represented by the angiogram image frame may be estimated using an ECG signal obtained at the same time as the angiogram. Alternatively, the position of the angiogram image frame along the cardiac cycle may be estimated based on the shape of the vessel within the angiogram image frame, which changes along the cardiac cycle, or by the relative position of any anatomical feature moving according the heart beats.

In step 150, the period of the cardiac cycle is estimated. The period of the cardiac cycle may be estimated based on ECG data, blood pressure measurement data (e.g. with sensor in blood pressure measurement cuff, sensor on an intravascular device) or based on the angiogram itself using the movement of the coronaries in the heart or the base frequency of the cross-sectional pulsation of vessels on the angiogram.

In step 160, a velocity curve representing the blood flow velocity within the vessel over the cardiac cycle is generated based on the period of the cardiac cycle, the estimated instantaneous blood flow velocity of each angiogram image frame, the confidence value of each angiogram image frame and the point in time within the cardiac cycle represented by or associated to each angiogram image frame.

In other words, every instantaneous blood flow velocity measurement, having a confidence value above a predetermined threshold, is aggregated to a one cardiac cycle long velocity curve, each instantaneous blood flow velocity measurement being positioned at the corresponding cardiac phase and taking into consideration the associated confidence value. For example, the confidence values may be binary and only those instantaneous blood flow velocity measurement having sufficiently clear contrast data may be aggregated into the curve. In this way, it is possible to reconstruct the blood flow velocity curve using a limited frequency of contrast pulses. Alternatively, the measurements may be combined using their confidence value as a relative weight in the combination, for example as a weighted average.

The method may be employed in situations where the contrast agent is provided to the vessel at an average frequency of 15Hz and below, for example at 1 Hz. In the example where the average frequency of injecting the contrast agent is 1Hz, the total injection protocol may be performed for 5 seconds. Assuming that the cardiac frequency is above 60 beats-per-minutes, the velocity curve may be estimated with at least 5 instantaneous blood flow velocity measurement points.

Put another way, using a low frequency pulsed injection of the contrast agent, instantaneous blood flow velocity values may be estimated alongside corresponding confidence values indicative of the amount of inherent contrast information contained in each angiogram image frame along the target vessel. The confidence values characterize the ability of a continuous velocity estimator to generate a unique velocity curve representing a cardiac cycle.

The above steps may be repeated after the subject has been provided with a stimulant or medicament in order to derive a relative index, such as coronary flow reserve (CFR) or renal indices, based on a comparison between the velocity curve and the stimulated velocity curve.

In addition, the processor may be further adapted to determine a vessel diameter for each angiogram image frame and determine a blood flow volume based on the vessel diameters and the velocity curve. In other words, the vessel diameter measured in the angiogram may be utilized to determine the volumetric flow rate instead of, or in addition to, the blood flow velocity.

Further, the processor may be adapted to obtain pressure measurement data from the vessel and calculate a vascular impedance based on the pressure data and the velocity curve. Put another way, based on the measured blood flow velocity curve and an additional blood pressure measurement, the vascular impedance may be determined. The blood pressure may be determined in any suitable way, such as via a fluid filled catheter or with a pressure sensor equipped guidewire.

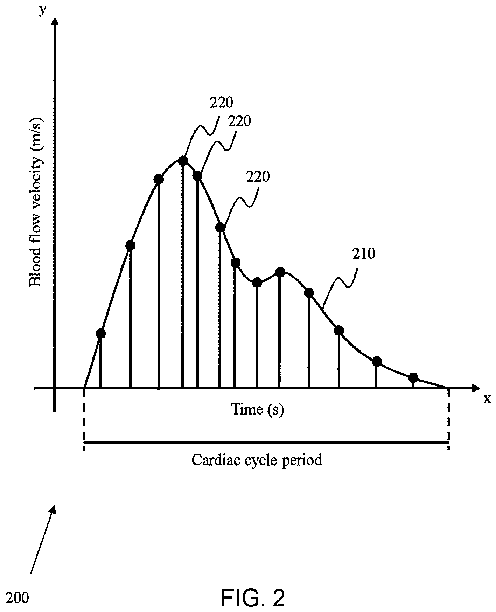

Figure 2 shows a graph 200 of blood flow velocity against time with a blood flow velocity curve 210 plotted over a cardiac cycle period.

Each point 220 along the blood flow velocity curve 210 represents an instantaneous blood flow velocity estimate derived from an angiogram image frame. The position of each point along the y-axis is adjusted based on the confidence value associated with each image frame corresponding to each point The points 220 are positioned along the x-axis based on the estimated point within the cardiac cycle represented by each angiogram image frame.

When the positions of the points 220 are finalized, the full blood flow velocity curve 210 is generated. The blood flow velocity curve may be further refined based on subsequent blood flow velocity measurements.

Figure 3 shows a schematic representation of a system 300 for obtaining a blood flow velocity measurement based on an angiogram obtained from a subject.

The system comprises a contrast agent injection device 310 adapted to inject a contrast agent 320 into a vessel 330 of the subject in bursts at a given average frequency, for example below 15Hz. During the intervention, a contrast agent is injected into the vessel of the subject. The contrast injection device produces a pulsed injection at a desired frequency, in a given injection pattern for a given duration. The contrast injection device may be mechanically operated in an automated manner or operated manually by a user, as high injection frequencies are not required. The pulse frequency may be desynchronized with respect to the cardiac frequency so that the produced contrast agent fronts cover different phases of the cardiac cycle.

The system further comprises an X-ray imaging device 340 adapted to obtain an angiogram of the vessel 330. The X-ray imaging system images the subject during the injection of contrast agent at a fixed framerate. The vessel segment where the blood flow velocity will be estimated must be within the field of view 345 of the X-ray imaging device.

The X-ray imaging device may obtain the angiogram at any given frame rate. For example X-ray imaging device may obtain the angiogram at a frame rate between 20Hz and 60Hz. For each injection of contrast agent into the vessel, the contrast agent will propagate along the vessel according to the blood flow within the vessel. Thus, each injection of contrast agent may generate a plurality of angiogram image frames including contrast data.

The system further includes a processor 350 adapted to perform the functions described above with respect to Figure 1.

In addition to the steps described above, the processor 350 may be further adapted to identify, based on the velocity curve, one or more of an optimal injection pattern for the contrast agent to be provided to the vessel and an optimal injection duration for the contrast agent to be provided to the vessel.

In addition to the injection pattern itself, i.e. the timing of the occurrences of the bursts of the contrast agent and the duration of said bursts, the real-time observation of the velocity curve, as it is generated, allows for the detection of when a sufficient number of blood flow velocity measurements have been obtained to generate a full velocity curve. Based on this determination, a signal may be triggered, automatically or through a signal sent to the user, or based on the user's own decision, to end the injection of the contrast agent. The distribution of the contrast agent injections may be an even distribution. Alternatively, the distribution of the contrast agent injections may be uneven in order to achieve an optimal distribution, such that at points on the velocity curve where the velocity gradient is higher, more blood flow velocity samples are obtained, as velocity estimation is more difficult in these areas.

The system may further comprise a dedicated feedback module 360 adapted to identify an optimal injection pattern or an optimal injection duration for the contrast agent to be provided to the vessel by way of the contrast agent injection device based on the velocity curve.

The feedback module 360 optimizes the injection pattern with respect to the current velocity curve reconstruction, to populate the velocity curve in an optimal manner. In the case of a manually controlled contrast injection device, a feedback signal may be provided to the user, for example by way of a display or a speaker, to guide the user to trigger the injection at the optimal instant or to cause the user to stop the injections when a satisfactory blood flow velocity curve is achieved. In the case of automatic mechanical injection, the feedback process may be automated.

By way of example, a convergence of the average blood flow velocity measurements may be used as an indicator that a sufficient number of contrast agent injections have been performed to reconstruct the full velocity curve. An alternative parameter that may be used to signal that the contrast agent injections may be stopped is when the percentage of the cardiac cycle that has been populated with blood flow velocity measurements exceeds a given threshold.

In a further aspect, a computer program is provided, wherein the computer program code means of the computer program are adapted to implement the methods according to the invention on any of the disclosed systems, when said computer program is run on a computer.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality.

A single processor or other unit may fulfill the functions of several items recited in the claims.

The mere fact that certain measures are recited in mutually different dependent claims does not indicate that a combination of these measures cannot be used to advantage.

A computer program may be stored/distributed on a suitable medium, such as an optical storage medium or a solid-state medium supplied together with or as part of other hardware, but may also be distributed in other forms, such as via the Internet or other wired or wireless telecommunication systems.

If the term "adapted to" is used in the claims or description, it is noted the term "adapted to" is intended to be equivalent to the term "configured to".

Any reference signs in the claims should not be construed as limiting the scope.

**Claims**

1. A processor (350) for use in a system (300) for obtaining a blood flow velocity measurement based on an angiogram obtained from a subject, wherein the processor is adapted to:

    obtain an angiogram of a vessel (330) of the subject, the angiogram comprising a plurality of angiogram image frames, wherein the angiogram is representative of the vessel including a contrast agent (320) therein, and wherein the contrast agent has been provided to the vessel in bursts, thereby providing contrast data within the angiogram;
    for each angiogram image frame of the angiogram that includes contrast data:

        estimate an instantaneous blood flow velocity based on the contrast data;
        calculate a confidence value of the angiogram image frame based on the contrast data; and
        estimate a point within a cardiac cycle represented by the angiogram image frame;

    estimate a period of the cardiac cycle; and
    generate a velocity curve representing the blood flow velocity within the vessel over the cardiac cycle based on the period of the cardiac cycle, the estimated instantaneous blood flow velocity of each angiogram image frame, the confidence value of each angiogram image frame and the point within the cardiac cycle represented by each angiogram image frame.

2. The processor (350) according to claim 1, wherein a frequency of bursts of the contrast agent (320) that has been provided to the vessel is desynchronized with the respect to the frequency of the cardiac cycle.

3. The processor (350) according to claim 1 or 2, wherein the bursts are separated by regular intervals.

4. The processor (350) according to claim 1 or 2, wherein the bursts are separated by irregular intervals.

5. The processor (350) according to any of the preceding claims, wherein a predetermined average frequency of bursts is less than or equal to 15Hz.

6. The processor (350) according to any of the preceding claims, wherein calculating the confidence value is further based on a noise level of the angiogram image frame or on the estimated instantaneous blood flow velocity.

7. The processor (350) according to any of the preceding claims, wherein estimating the period of the cardiac cycle is based on one or more of: the angiogram, ECG data and blood pressure measurement.

8. The processor (350) according to any of the preceding claims, wherein the processor is further adapted to identify, based on the velocity curve, one or more of:

    an optimal injection pattern for the contrast agent to be provided to the vessel; and
    an optimal injection duration for the contrast agent to be provided to the vessel.

9. The processor (350) according to any of the preceding claims, wherein the processor is further adapted to:

    obtain blood pressure measurement data from the vessel; and
    calculate a vascular impedance based on the pressure measurement data and the velocity curve.

10. The processor (350) according to any of the preceding claims, wherein the processor is further adapted to:

    obtain a second angiogram of a vessel of the subject, the second angiogram having been acquired after a stimulus was provided to the subject, the second angiogram comprising a plurality of second angiogram image frames, wherein the second angiogram is representative of the vessel including a contrast agent therein, and wherein the contrast agent has been provided to the vessel in bursts, thereby providing contrast data within the angiogram;
    for each second angiogram image frame of the angiogram that includes contrast data:

        estimate a stimulated instantaneous blood flow velocity based on the second angiogram image frame;
        calculate a stimulated confidence value of the second angiogram image frame based on the contrast data; and
        estimate a point within a cardiac cycle represented by the second angiogram image frame;

    estimate a period of the cardiac cycle;
    generate a stimulated velocity curve for the cardiac cycle based on the period of the cardiac cycle, the estimated stimulated instantaneous blood flow velocity of each second angiogram image frame, the stimulated confidence value of each second angiogram image frame and the point within the cardiac cycle represented by each second angiogram image frame;

compare the velocity curve and the stimulated velocity curve; and

calculate a relative index based on the comparison.

11. The processor (350) according to any of the preceding claims, wherein the processor is further adapted to:

determine a vessel diameter for on each angiogram image frame; and

determine a blood flow volume based on the vessel diameters and the velocity curve.

12. A system (300) for obtaining a blood flow velocity measurement based on an angiogram obtained from a subject, the system comprising:

a contrast agent injection device (310) adapted to inject a contrast agent (320) into a vessel (330) of the subject in bursts;

an X-ray imaging device (340) adapted to obtain an angiogram of the vessel; and

a processor (350) according to any of the preceding claims.

13. A system (300) as claimed in claim 12, wherein the contrast agent injection device is either:

operated automatically based on the optimal injection pattern or the optimal injection duration identified by the processor, or

manually by a user, and wherein the system further comprises a display adapted to display information related to the optimal injection pattern or the optimal injection duration to the user.

14. A method (100) of ascertaining blood flow velocity based on an angiogram obtained from a subject, comprising:

obtaining an angiogram of a vessel (110) of the subject, the angiogram comprising a plurality of angiogram image frames, wherein the angiogram is representative of the vessel including a contrast agent (320) therein, and wherein the contrast agent has been provided to the vessel in bursts, thereby providing contrast data within the angiogram;

for each angiogram image frame of the angiogram that includes contrast data:

estimating an instantaneous blood flow velocity (120) based on the contrast data;

calculating a confidence value (130) of the angiogram image frame based on the contrast data; and

estimating a point within a cardiac cycle (140) represented by the angiogram image frame;

estimating a period of the cardiac cycle (150); and

generating a velocity curve (160) representing the blood flow velocity within the vessel over the cardiac cycle based on the period of the cardiac cycle, the estimated instantaneous blood flow velocity of each angiogram image frame, the confidence value of each angiogram image frame and the point within the cardiac cycle represented by each angiogram image frame.

15. A computer program comprising computer program code means, wherein the computer program code means of the computer program are adapted to implement the method according to claim 14 on the system of claim 12, when said computer program is run on a computer.

Obtain an angiogram of a vessel — 110

For each angiogram image frame

Estimate an instantaneous blood flow velocity — 120

Calculate a confidence value — 130

Estimate a point within a cardiac cycle — 140

Estimate a period of the cardiac cycle — 150

Generate a velocity curve — 160

100

FIG. 1

FIG. 2

FIG. 3

Europäisches Patentamt
European Patent Office
Office européen des brevets

# EUROPEAN SEARCH REPORT

Application Number

EP 20 29 0069

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | WO 2007/069166 A2 (KONINKL PHILIPS ELECTRONICS NV [NL]; PHILIPS CORP [US] ET AL.) 21 June 2007 (2007-06-21) <br> * page 6, line 22 - page 16, line 17 * <br> ----- | 1-15 | INV. <br> A61B6/00 |
| A | US 2017/325770 A1 (EDIC PETER MICHAEL [US] ET AL) 16 November 2017 (2017-11-16) <br> * paragraph [0026] - paragraph [0106] * <br> ----- | 1-15 | |

TECHNICAL FIELDS
SEARCHED      (IPC)

A61B

The present search report has been drawn up for all claims

| Place of search | Date of completion of the search | Examiner |
|---|---|---|
| Munich | 3 March 2021 | Hooper, Martin |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 20 29 0069

This annex lists the patent family members relating to the patent documents cited in the above-mentioned European search report.
The members are as contained in the European Patent Office EDP file on
The European Patent Office is in no way liable for these particulars which are merely given for the purpose of information.

03-03-2021

| Patent document cited in search report | | Publication date | Patent family member(s) | | Publication date |
|---|---|---|---|---|---|
| WO 2007069166 | A2 | 21-06-2007 | CN | 101330872 A | 24-12-2008 |
| | | | EP | 1962688 A2 | 03-09-2008 |
| | | | JP | 5236489 B2 | 17-07-2013 |
| | | | JP | 2009519082 A | 14-05-2009 |
| | | | US | 2008319309 A1 | 25-12-2008 |
| | | | WO | 2007069166 A2 | 21-06-2007 |
| US 2017325770 | A1 | 16-11-2017 | NONE | | |

EPO FORM P0459

For more details about this annex : see Official Journal of the European Patent Office, No. 12/82

## REFERENCES CITED IN THE DESCRIPTION

**Non-patent literature cited in the description**

- Fractional Flow Reserve Versus Angiography for Guiding Percutaneous Coronary Intervention in Patients With Multivessel Coronary Artery Disease. **PIJLS, NICO H. J. et al.** Journal of the American College of Cardiology. Elsevier Inc, July 2010, vol. 56, 177-84
- **KHANMOHAMMADI et al.** Automatic Estimation of Coronary Blood Flow Velocity Step 1 for Developing a Tool to Diagnose Patients With Micro-Vascular Angina Pectoris. *Frontiers in Cardiovascular Medicine,* January 2019
- **BONNEFOUS.** Quantification of arterial flow using digital subtraction angiography. *Med. Phys.,* October 2012, vol. 39 (10
- **HOLDSWORTH, D. W. et al.** Quantitative Angiographic Blood-Flow Measurement Using Pulsed Intra-Arterial Injection. *Medical Physics,* October 1999, vol. 26 (10), 2168-75
- **SHAW, C. G. ; D. B. PLEWES.** Pulsed-Injection Method for Blood Flow Velocity Measurement in Intraarterial Digital Subtraction Angiography. *Radiology,* August 1986, vol. 160 (2), 556-59
- **SHAW, C. G. ; D. B. PLEWES.** Pulsed-Injection Method for Blood Flow Velocity Measurement in Intraarterial Digital Subtraction Angiography. *Radiology,* August 1986, vol. 160 (2), 556-59